# EUROPEAN PATENT APPLICATION

(11) **EP 4 775 168 A1**
(43) Date of publication of application: **15.07.2026**
(21) Application number: 25151781.9
(22) Date of filing: 14.01.2025
(51) Int. Cl.: A61B 34/30, A61B 46/10

(54) **INSTRUMENT-ADAPTER ASSEMBLY**

(71) Applicant: Microsure B.V., 5692 EA Son (NL)
(72) Inventor: Boerma, Erik Niels, 5616 BS Eindhoven (NL)
(74) Representative: WENDE IP GbR

(57) **Abstract**

The present invention provides an instrument-adapter assembly (200; 300) for an end-effector system, comprising a surgical instrument (202; 302) including: a grip portion (204; 304), a tip portion (206; 306), and a predefined workpoint (W), and a surgical instrument adapter (208; 308), configured to hold and operate the surgical instrument (202; 302), comprising: a first movable arm (210; 310); a second movable arm (212; 312), and a base portion (214), the first movable arm (210; 310) and the second movable arm (212; 312) being hingedly connected through said base portion (214). The surgical instrument (202; 302) comprises a first coupling and alignment means (216; 316), formed on an external surface of the surgical instrument (202; 302). The surgical instrument adapter (208; 308) comprises a second coupling and alignment means (218; 318), configured for engagement with said first coupling and alignment means (216; 316), the second coupling and alignment means (218; 318) being formed on an internal surface of the first movable arm (210; 310) and/or the second movable arm (212; 312) of the surgical instrument adapter (208; 308).

## Description

The present invention belongs to the technical field of robotic systems, methods, and techniques for use in surgery, microsurgery or super-microsurgery.

In particular, the present invention relates to an instrument-adapter assembly for an end-effector system for a surgical, microsurgical, or super-microsurgical robot arm.

The invention further relates to an end-effector system for a surgical, microsurgical or super-microsurgical robot arm, said end-effector system *inter alia* comprising an instrument-adapter assembly as defined above.

Still further, the invention provides a surgical, microsurgical or super-microsurgical robot arm, said robot arm comprising an end-effector system as defined above.

In a non-limiting example, the robot arm can be used to perform anastomoses.

Different kinds of surgical instruments are usually necessary when a surgical, microsurgical or super-microsurgical operation is carried out on a patient.

These surgical instruments are often configured to be manually held and manipulated by an operator, e.g., a surgeon.

Alternatively, said surgical instruments can be manipulated through a robotic system comprising one or more robot arms.

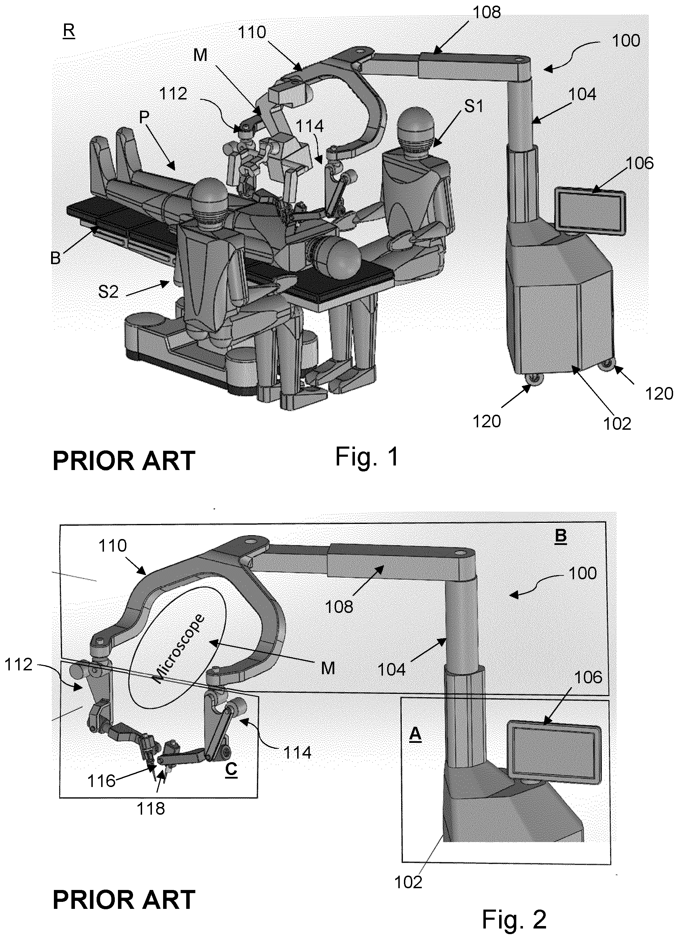

An exemplary configuration of a robotic system for use in surgery, microsurgery or super-microsurgery is shown in **Figs. 1-2**.

The use of a robotic system facilitates accurate movements with micrometer precision without substantial tremor, thereby superseding the limitations of human hand manipulation.

Here, the use of an adapter is required to provide an interface between the surgical instrument and the end-effector.

Surgical instrument adapters for use in robotic systems of this kind are known in the art.

An exemplary surgical instrument adapter is described in WO2022233585A1, filed in the name of the same applicant.

In the configuration according to WO2022233585A1 (illustrated in **Fig. 3**) the surgical instrument adapter comprises a clip for holding at least one surgical instrument in a predefined orientation with respect to the end-effector. The surgical instrument adapter transmits a movement of a tip portion of first and second fingers of the end-effector to the at least one surgical instrument, to manipulate said at least one surgical instrument.

An improved surgical instrument adapter is disclosed in EP23207208, filed in the name of the same applicant.

When a surgical instrument is manually operated, the surgeon holds the instrument with his/her hand, in a pen-like fashion.

During operation, the rear of the instrument rests on the hand of the surgeon holding a grip portion of the instrument between the index-finger and the thumb.

Fingers are soft and, while carrying out a surgical operation, covered in a glove.

To prevent slipping, surgical instruments configured for manual operation are typically provided with a knurl, formed on the grip portion.

When a surgical instrument configured for manual operation needs to be interfaced with a robotic system (e.g., the system shown in **Fig. 1-2**), some difficulties may rise.

For instance, the knurl can tear the sterile drape covering the end-effector.

Also, contact with the rear part of the surgical instrument may hamper the instrument's movement.

The use of an instrument adapter as described in the foregoing may help to at least partially address these issues.

The instrument adapter is placed over the sterile drape, in particular to prevent contact between the knurl and the sterile drape, which may tear or otherwise damage the sterile drape.

The adapter has an instrument-specific geometry that prevents movement hampering.

However, correct alignment of the surgical instrument with respect to the adapter is often difficult to obtain, especially when a surgical instrument designed for manual operation is used with a robotic system.

To date, only partial solutions have been provided to facilitate correct positioning of a surgical instrument designed for manual operation with respect to a surgical instrument adapter (and, in turn, to the end effector).

For instance, in case a needle holder configured for manual operation is to be used with a robotic system, a known solution consists in the provision of a lock, provided in the adapter and configured to latch onto an existing pin formed in the instrument.

Here, the lock snaps around the pin, thus aligning the instrument only in the direction of its axis.

This solution has the drawback that usability issues may occur during placement and/or removal of the instrument into/from the adapter.

A further drawback of this solution is that it relies on the position of the pin, which has a function that does not relate to its position. The pin has a length that is tuned for its function. This means that a series of instruments can be produced having a respective pin located in different positions.

Indeed, this is not prejudicial to manual operation of the instrument.

However, if the pin is used for alignment purposes, thus assuming a stable position for the pin, this could pose a usability risk.

In particular, when formed in a new position, the pin may still fit with the instrument, but cause the instrument to be misaligned.

Similarly, in case a forceps-type instrument configured for manual operation is to be used with a robotic system, a known solution consists in the provision of an endstop, arranged at the rear portion of the instrument. All length variations of the instruments will end up at the tip because of this.

Nonetheless, the instrument is not fixed in that position. To the contrary, this only represents a way to initially align the instrument. Here, it is assumed that the instrument does not implement any movement along its axis.

Also, no means such as a pin is formed on the instrument.

Moreover, this solution may lead to less accurate tip movements, as they deviate from the workpoint of the robot, i.e. the origin around which the instrument is positioned when operated.

The workpoint is located just inside the instrument's tips.

When the instrument is displaced, or its tips are in a deviating position because of instrument length differences, the robot will rotate (pose) the instrument around a different point than expected by the surgeon.

Yet another drawback is that, when a surgical instrument designed for manual operation is interfaced with a robotic system, it is difficult to prevent slipping of the instrument along its axis during use.

Since the instrument is held on both sides of the grip portion, the alignment problem is present at both sides. This means that misalignment can also occur in different amounts, causing the instrument's axis to be misaligned with the axis of the end-effector of the robot arm, again leading to an offset between the workpoint and the tip of the instrument (in this case not along its axis, but perpendicular to that axis).

This may potentially hinder the overall functionality.

Hence, there is the need for an improved solution allowing the operator, e.g., a surgeon, to easily, reliably, and intuitively interface a surgical instrument with the end effector of a robot arm and facilitating alignment of the surgical instrument with respect to the end-effector, even in case a surgical instrument designed for manual operation is used.

In view of the above, it is an object of the present invention to provide an instrument-adapter assembly allowing an operator, e.g., a surgeon, to easily, reliably, and intuitively obtain correct alignment of a surgical instrument with respect to the end-effector of a surgical robot arm, even in case a surgical instrument designed for manual operation is used.

The above-specified object is achieved by the provision of an instrument-adapter assembly as defined in claim 1.

According to the invention, an instrument-adapter assembly for an end-effector system comprises:
a surgical instrument comprising a grip portion, a tip portion, distally arranged with respect to said grip portion, and a predefined workpoint, and
a surgical instrument adapter, configured to hold the surgical instrument and
operate the surgical instrument by transmitting movement from an end-effector of the end-effector system to the surgical instrument, said surgical instrument adapter comprising:
   a first movable arm;
   a second movable arm, and
   a base portion,
the first movable arm and the second movable arm being hingedly connected through said base portion,
wherein the surgical instrument comprises a first coupling and alignment means, formed on an external surface of the surgical instrument;
wherein the surgical instrument adapter comprises a second coupling and alignment means, configured for engagement with said first coupling and alignment means, to facilitate positioning and maintain correct placement of the surgical instrument with respect to the surgical instrument adapter, and
wherein the second coupling and alignment means is formed on an internal surface of the first movable arm and/or the second movable arm of the surgical instrument adapter.

The present invention provides an instrument-adapter assembly.

In particular, the instrument-adapter assembly is configured for use with an end-effector system for a surgical, microsurgical or super-microsurgical robot arm.

In a non-limiting example, the robot arm can be used to perform anastomoses.

The instrument-adapter assembly comprises a surgical instrument.

The surgical instrument is adapted for use in surgical, microsurgical or super-microsurgical procedures.

In particular, said surgical instrument is designed to be manually operated.

The surgical instrument comprises a grip portion.

Since the surgical instrument is designed for manual operation, the grip portion may include a knurled portion.

The surgical instrument further comprises a tip portion.

The tip portion is distally arranged with respect to the grip portion.

In this context, the term "distal" is used to denote a side that is closer to the patient and farther from the end-effector during use.

The surgical instrument further has a predefined workpoint.

In this context, the term "workpoint" is used to denote a position along the instrument axis at which the instrument is intended to interact with an object, e.g. a surgical needle, a suture, or the like.

In case of scissors-type or forceps-type surgical instruments, this point usually is located just inside the instrument tips, e.g., at a distance of 1 mm from the distal end of the instrument.

This point is used by the surgical robot as the origin for positioning and posing the instrument.

The assembly further comprises a surgical instrument adapter.

The surgical instrument adapter is configured to hold the surgical instrument and operate the surgical instrument by transmitting movement from an end-effector of the end-effector system to the surgical instrument.

Otherwise stated, the surgical instrument adapter provides an interface between the surgical instrument and the end-effector of the robot arm.

The surgical instrument adapter comprises a first movable arm.

The surgical instrument adapter further comprises a second movable arm.

The surgical instrument adapter further comprises a base portion.

The first and second movable arms are hingedly connected through said base portion.

Hence, the first and second movable arm are able to perform an opening/closing movement with respect to each other.

The surgical instrument comprises a first coupling and alignment means.

The first coupling an alignment means are formed on an external surface of the surgical instrument.

The surgical instrument adapter comprises a second coupling and alignment means.

The second coupling and alignment means is configured for engagement with the first coupling and alignment means.

This allows to facilitate positioning and maintain correct placement/alignment of the surgical instrument with respect to the surgical instrument adapter.

The second coupling and alignment means is formed on an internal surface of the first movable arm and/or the second movable arm of the surgical instrument adapter.

The invention is based on the basic idea that, by the provision of first and second coupling and alignment means as defined above, correct alignment of the surgical instrument with respect to the end-effector can be easily, intuitively, and reliably accomplished, even in case a surgical instrument designed for manual operation is used. Hence, the risk of incorrect placement/alignment of the surgical instrument can be largely avoided.

Also, the surgical instrument can be reliably maintained in place during operation, without prejudice to movement and/or maneuverability of the instrument.

In the state of the art, surgical instrument adapters are often provided with clips, configured to hold the surgical instrument in place.

These clips need to be tight in order to prevent instrument displacement.

With the solution according to the invention, this tightness can be decreased, making placement of the instrument easier for the operator, e.g., a surgeon.

Conveniently, the coupling and alignment means can be formed on the grip portion of the surgical instrument.

This provides an easy and intuitive solution allowing the operator, e.g., a surgeon, to correctly position the surgical instrument with respect to the surgical instrument adapter, even in case a surgical instrument designed for manual operation is used.

Conveniently, the first coupling and alignment means and the second coupling and alignment means may be configured for shape-coupling.

This solution allows to define a fixed orientation for the instrument, which in some cases could be appropriate.

For instance, when a bent instrument is used, it can be placed with its tip always facing a certain direction with respect to the orientation of the robot. This allows for the definition of a workpoint that is offset from the instrument axis to further improve accuracy.

Conveniently, the first coupling and alignment means can be formed on the grip portion of the surgical instrument, arranged at a predefined distance from the predefined workpoint.

This allows obtaining an optimized solution for correct positioning of the surgical instrument, regardless of the kind and/or length variation of the instrument.

Said predefined distance shall be determined on a case-by-case basis, depending on the characteristics, e.g., length, of the surgical instrument.

Preferably, the first coupling and alignment means comprises at least one hole.

Here, the second coupling and alignment means comprises at least one corresponding pin, configured for engagement with the at least one hole.

This allows providing a simple and reliable solution enabling for correct positioning of the surgical instrument, even when a surgical instrument adapted for manual operation is used with a robotic system.

Also, the engagement between the pin and the corresponding hole provides a haptic feedback confirming to the operator, e.g., a surgeon, that the surgical instrument is correctly positioned and ready for use.

Even more preferably, the first coupling and alignment means comprises a first hole and a second hole.

In particular, said first and second holes are each formed on a respective side of the surgical instrument.

Correspondingly, the second coupling and alignment means comprises a first pin, for engagement with the first hole and a second pin, configured for engagement with the second hole.

Accordingly, the surgical instrument can be correctly aligned on both sides, and can be reliably maintained in the correct position/orientation during operation.

Surgical instruments configured for manual operation are often handmade (or, at least, hand-finished).

Accordingly, there is a large variation in physical dimensions, e.g., length, among surgical instruments.

One or more holes can be formed in the instrument at the end of the manufacturing process (or even at a later stage).

This allows obtaining an improved level of accuracy compared to solutions where a predefined geometry for correct alignment is provided/added at the beginning of the manufacturing process, as in the case of known instruments designed for use with a robot system (and not for manual operation).

Also, one or more holes (e.g., two) can be formed in the instrument without hampering its use and/or maneuverability.

By contrast, the instrument would still be suitable even for manual operation.

Still further, the presence of the one or more holes formed on the instrument's body is not prejudicial to cleanability of the instrument, and does not interfere with other features (e.g., other holes, pins, or the like) that may be present on the instrument's body.

By forming a hole on an external surface on both sides of the surgical instrument, the holes being arranged at the same position and having the same size, it is also possible to differentiate left and right handle for instrument with a tip outside the instrument axis.

This can also be achieved by acting on the diameter and/or position of the holes formed in each instrument handle.

This allows for easy placement of the instrument in a dedicated orientation.

In one configuration, the first hole and corresponding first pin and the second hole and corresponding second pin may have different sizes.

In this configuration, the first hole and the second hole may be offset with respect to each other by a predefined distance.

The first pin and second pin are correspondingly offset with respect to each other by the same distance.

Here, the robot arm may have a fixed orientation and include a plurality of holes, and the surgical instrument must be correspondingly inserted.

Although not frequent, it might be the case that the wrong surgical instrument is unwantedly inserted into a certain surgical instrument adapter.

This may hamper operation of the surgical instrument.

The above-described solution allows preventing this undesired occurrence.

Also, by the provision of different pin/hole combinations, it is possible to increase the number of successful matching combinations between a surgical instrument and corresponding surgical instrument adapter.

In an alternative configuration, the first hole and corresponding first pin and the second hole and corresponding second pin may have the same size.

Here, the distance between the workpoint of the instrument and the first hole and corresponding first pin, and the distance between the workpoint of the instrument and the second hole and corresponding second pin may be the same.

The surgical instrument can be a forceps-type surgical instrument.

Alternatively, the surgical instrument can be a scissors-type surgical instrument.

As a non-limiting example, the surgical instrument can be one among a needle holder, a forceps, a dilator, scissors, or a combination of a needle holder with scissors in a single instrument, configured to be manually operated by the user.

Forceps-type and scissors-type surgical instruments include a first instrument arm and a second instrument arm, configured to perform an opening/closing movement with respect to each other.

Here, the first hole is formed on the external surface of the grip portion part formed on the first instrument arm, and the second hole is formed on the external surface of the grip portion part formed on the second instrument arm.

The present invention further provides an end-effector system.

The end-effector system is configured for use with a surgical, microsurgical or super-microsurgical robot arm.

In a non-limiting example, the robot arm can be used to perform anastomoses.

The end-effector system comprises an end- effector.

The end-effector comprises an end-effector base.

The end-effector further comprises a first movable finger.

The end-effector further comprises a second movable finger.

The first and second movable fingers extend distally from the end-effector base.

The end-effector system further comprises a sterile drape.

The sterile drape is configured to cover the end-effector.

This allows maintaining the sterile barrier during use.

The end-effector system further comprises an instrument-adapter assembly as defined in the foregoing.

The surgical instrument adapter of the instrument-adapter assembly is arranged over the sterile drape.

Also, the surgical instrument adapter is configured to transmit a movement of a tip portion of the first and second movable fingers of the end-effector to the surgical instrument to be operated through the robot arm.

The present invention further provides a surgical, microsurgical or super-microsurgical robot arm.

In a non-limiting example, the robot arm can be used to perform anastomoses.

The robot arm comprises an end-effector system as defined in the foregoing.

Further advantages of the present invention will now be disclosed in connection with the drawings, where:
- **Fig. 1**: shows a robotic system for use in surgery, microsurgery or super-microsurgery according to the prior art, in an operative state;
- **Fig. 2**: shows a detail of the surgical robotic system of **Fig. 1**, where different sections of the robotic system, respectively denoted with letters **A**, **B**, **C**, are identified;
- **Fig. 3**: shows an end-effector system provided with a surgical instrument adapter according to the prior art, in particular as described in WO2022233585A1. Here, a surgical instrument is mounted to the adapter.
- **Fig. 4**: shows and instrument-adapter assembly according to a first embodiment of the invention;
- **Fig. 5**: shows an exemplary surgical instrument for use in the instrument-adapter assembly of **Fig. 1**, here a surgical needle holder;
- **Fig. 6**: shows a different view of the surgical instrument of **Fig. 5**;
- **Fig. 7**: shows a cross-section view of the surgical instrument of **Fig. 5**. Here, first coupling and alignment means formed in the instrument can be seen. In particular, said first coupling and alignment means include a first hole and a second hole, each formed on the external surface of a respective arm of the instrument;
- **Fig. 8**: shows a partial, detail view of the instrument-adapter assembly of **Fig. 1**, in particular showing the engagement between the first coupling and alignment means formed in the instrument (first and second holes, as illustrated in **Fig. 7**) and corresponding second coupling and alignment means, here in the shape of first and second pins, formed in the instrument adapter;
- **Fig. 9**: shows a partial, detail view of an instrument-adapter assembly according to another embodiment of the invention, in particular showing the engagement between the first coupling and alignment means formed in the instrument (here, first and second holes, as shown in **Fig. 11** discussed below) and corresponding second coupling and alignment means (here, first and second pins), formed in the instrument adapter;
- **Fig. 10**: shows an exemplary surgical instrument for use in the instrument-adapter assembly of **Fig. 9**, here a forceps;
- **Fig. 11**: shows a cross-section view of the surgical instrument of **Fig. 10**. Here, the first coupling and alignment means formed in the instrument can be seen. Similar as above, said first coupling and alignment means include a first hole and a second hole, each formed on the external surface of a respective arm of the instrument.

**Fig. 1** shows an example of a robotic system for use in surgery, microsurgery or super-microsurgery.

Said exemplary system is denoted with 100.

Here, the system 100 is shown in an operating state.

A first surgeon S1 and a second surgeon S2 are located at a respective side of an operating bed B to perform a surgical, microsurgical or super-microsurgical operation on a patient P, laying on operating bed B.

The robotic system 100 includes a base station 102.

The surgical robotic system 100 further includes a base column 104, here in the shape of a cylindrical pillar.

In the shown example, the base station 102 conveniently includes a display module 106 for displaying information to one or more operators, e.g., the surgeons S1, S2 or other personnel that is present in the operating room OR.

Also, the display module 106 may be used to define a user interface allowing one or more operators to provide a user input.

The base station 102 comprises a plurality of wheels 120 for ease of placement of the robotic system 100 at a desired location within the operating room OR.

In the shown example, the robotic system 100 is equipped with a suspension arm 108, a fork-like element 110, a first surgical, microsurgical or super-microsurgical robot arm 112, and a second surgical, microsurgical or super-microsurgical robot arm 114.

Here, the suspension arm 108 is horizontally arranged.

Also, the suspension arm 108 has two parts allowing to adjust its length by telescopically moving said parts relatively to each other.

It is also possible that a different means is used for length adjustment of the suspension arm 108, e.g., a SCARA-mechanism, hinge mechanisms, or the like.

The suspension arm 108 is connected to the base column 104.

The suspension arm 108 carries the fork-like element 110 (e.g., a bracket).

As shown in detail in **Fig. 2**, the fork-like element 110 carries the first surgical, microsurgical or super-microsurgical robot arm 112 and the second surgical, microsurgical or super-microsurgical robot arm 114.

Each of the first and second robot arms 112, 114 is connected to an end-effector system carrying a surgical instrument 116, 118 (**Fig. 2**).

The surgical instruments 116, 118 may be of the same kind, or may be different from each other.

The suspension arm 108 is configured to rotate about the longitudinal axis of the base column 104, to allow desired positioning of the fork-like element 110 and the robot arms 112, 114 with respect to the surgical site.

Similarly, the fork-like element 110 is configured to rotate about an axis of the suspension arm 108, i.e., parallel to the longitudinal axis of the base column 104.

The robotic system 100 may be adapted for use with a conventional microscope M, as shown in **Fig. 2**.

Alternatively, a fully digital microscope (not shown) or a so-called hybrid microscope (not shown) can be used. In such a case, one or more surgeons will be located at a separate console, and a robot trolley will be provided at the position of one of the surgeons S1, S2.

The surgical robotic system 100 provides a large patient side setup, this meaning that no assembly is required after draping.

**Fig. 2** shows different sections A, B, C of the surgical robotic system 100.

In section A, there is no movement and everything is thus stationary.

The display means 106 (included in section A) can be activated by one or more operators.

In section B, there is some movement during the surgery, in order to correctly reposition the surgical instruments 116, 118 over the patient P.

As shown in **Fig. 2**, a space is defined between the surgical robotic arms 112, 114 for a microscope (not illustrated but only symbolized in **Fig. 2**).

Section C includes, inter alia, the end-effector.

Here, several movements occur in the course of the surgical procedure.

In this context, the functions of the end-effector are, inter alia, to actuate the surgical instruments 116, 118, provide a mounting interface, and rotate around the instrument axis.

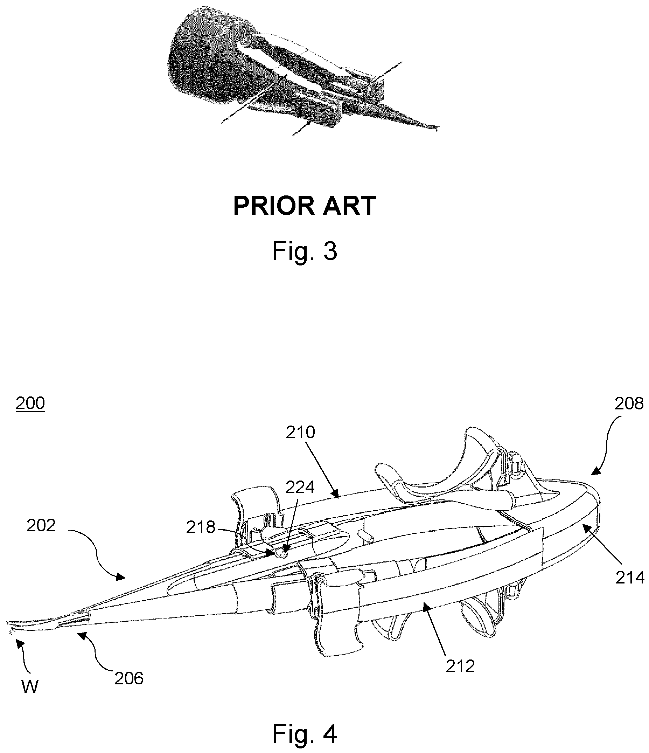

**Fig. 4** shows an instrument-adapter assembly 200 according to a first embodiment of the invention.

The instrument-adapter assembly 200 is configured for use with an end-effector system (not shown).

The end-effector system is configured for use with a surgical, microsurgical or super-microsurgical robot arm (not shown).

In a non-limiting example, the robot arm can be used to perform anastomoses.

The instrument-adapter assembly comprises a surgical instrument 202 and a surgical instrument adapter 208.

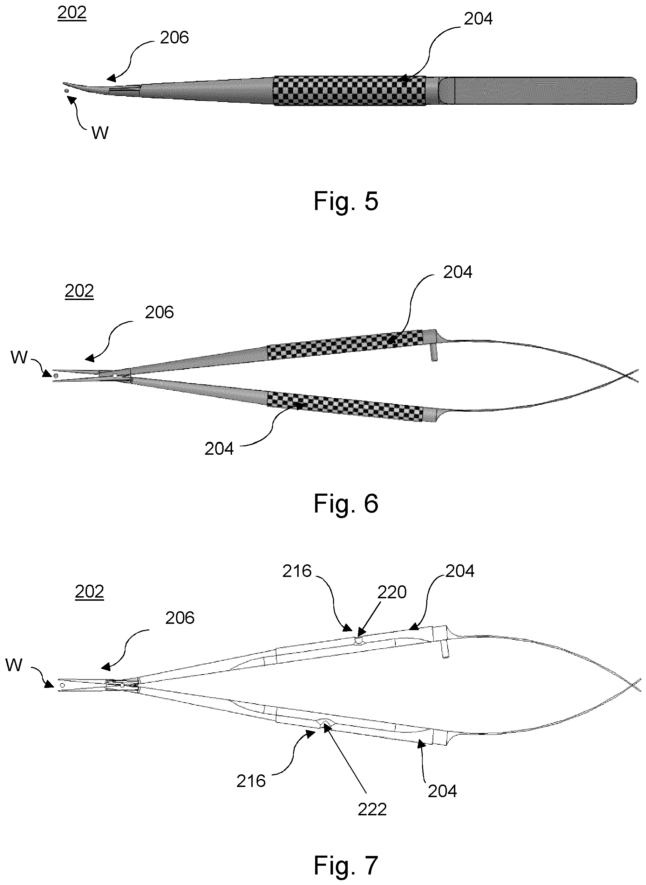

In the shown embodiment, the surgical instrument is a surgical needle holder, as illustrated in **Figs. 5-7**.

The surgical instrument 202, here a surgical needle holder, is adapted for use in surgical, microsurgical or super-microsurgical procedures.

The surgical instrument 202 is configured for manual operation.

The surgical instrument 202 comprises a grip portion 204, a tip portion 206, distally arranged with respect to said grip portion 204.

The surgical instrument has a predefined workpoint W (**Figs. 5-7**).

In this context, the term "distal" is used to denote a side that is closer to the patient and farther from the end-effector during use.

The surgical instrument adapter 208 is configured to hold the surgical instrument 202 and operate the surgical instrument 202 by transmitting movement from an end-effector of the end-effector system to the surgical instrument 202.

That is, the surgical instrument adapter 208 provides an interface between the surgical instrument 202 and the end-effector.

The surgical instrument adapter 208 comprises a first movable arm 210, a second movable arm 212, and a base portion 214.

Here, the first and second movable arms 208, 210 are hingedly connected through the base portion 208.

The surgical instrument 202 comprises a first coupling and alignment means 216.

The first coupling and alignment means 216 are formed on an external surface of the surgical instrument 202.

In the present embodiment, the first coupling and alignment means 216 is formed on the grip portion 204 of the surgical instrument 204, arranged at a predefined distance from the predefined workpoint W.

Said predefined distance shall be determined on a case-by-case basis, depending on the characteristics, e.g., length, of the surgical instrument 202.

The surgical instrument adapter 208 comprises a second coupling and alignment means 218.

The second coupling and alignment means 218 is configured for engagement with said first coupling and alignment means 216.

This allows to facilitate positioning and maintain correct placement of the surgical instrument 202 with respect to the end-effector, even in case a surgical instrument designed for manual operation is used.

The second coupling and alignment means 218 is formed on an internal surface of the first movable arm 210 and/or the second movable arm 212 of the surgical instrument adapter 208.

In the present embodiment, the first coupling and alignment means 216 and the second coupling and alignment means 218 are configured for shape-coupling.

The first coupling and alignment means 216 comprises at least one hole 220, 222, , and the second coupling and alignment means 218 comprises at least one corresponding pin 224, 226, configured for engagement with the at least one hole 220, 222.

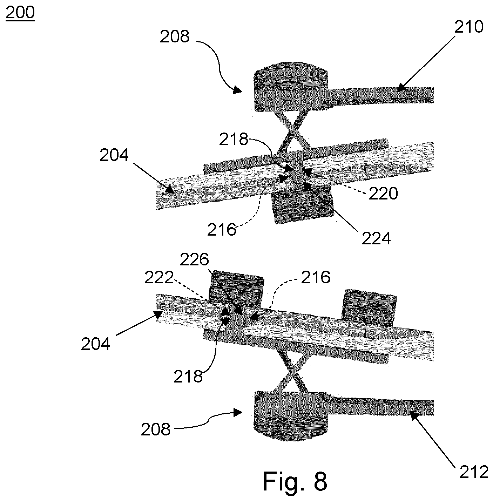

The engagement between the first coupling and alignment means 216 and the second coupling and alignment means 218 is shown in the detail view provided in **Fig. 8**.

In the present embodiment, the first coupling and alignment means 216 comprises a first hole 220 and a second hole 222, each formed on a respective side of the surgical instrument 202, as shown in **Fig. 7**.

As mentioned, on the shown embodiment, the surgical instrument 202 is a needle holder, including a first instrument arm and a second instrument arm (as best seen in **Figs. 6-7**), configured to perform an opening/closing movement with respect to each other.

Here, the first hole 220 is formed on the external surface of the grip portion part formed on the first instrument arm, while the second hole 222 is formed on the external surface of the grip portion part formed on the second instrument arm (**Fig. 7**).

The second coupling and alignment means 218 comprises a first pin 224, configured to engage with the first hole 220, and a second pin 226, configured for engagement with the second hole 222 (**Fig. 8**).

In the present embodiment, the first hole 220 and corresponding first pin 224 and the second hole 222 and corresponding second pin 226 have different sizes (**Fig. 8**).

Also, in the present embodiment, the first hole 220 and the second hole 222 are offset with respect to each other by a predefined distance, and the first pin 224 and second pin 226 are correspondingly offset with respect to each other by the same distance (**Fig. 8**).

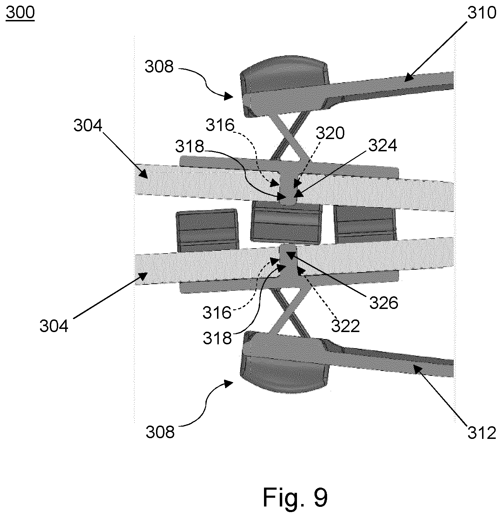

**Fig. 9** shows a partial, detail view of an instrument-adapter assembly 300 according to a further embodiment of the invention.

The instrument-adapter assembly 300 includes features that are similar to that of the instrument-adapter assembly 200 according to the first embodiment described in the foregoing.

In particular, similar as above, the instrument-adapter assembly 300 comprises a surgical instrument 302 comprising a grip portion 304, and a tip portion 306, distally arranged with respect to said grip portion 304.

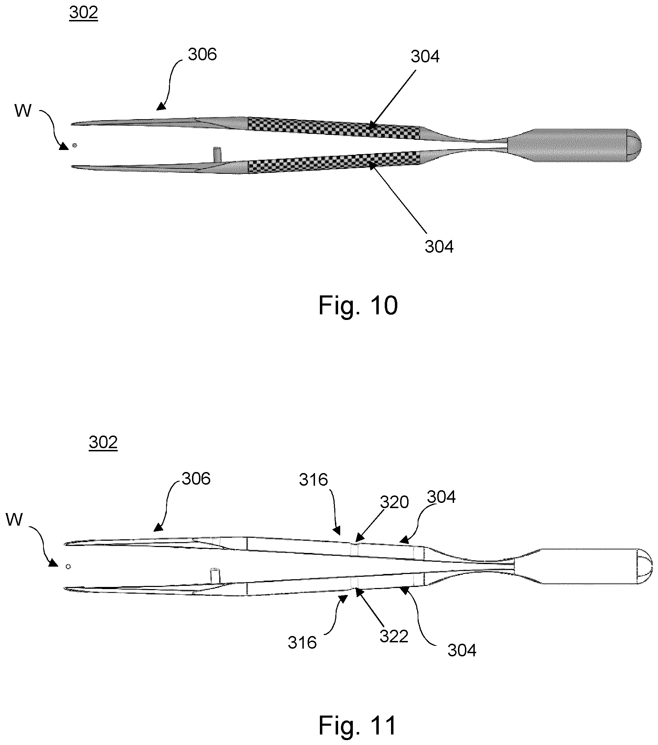

The surgical instrument 302 as a predefined workpoint W, as defined in the foregoing (**Figs 10-11**).

The instrument-adapter assembly further comprises a surgical instrument adapter 308 comprising a first movable arm 310, a second movable arm 312, and a base portion (not shown), hingedly connecting the first and second movable arms 310, 312.

The instrument adapter 308 operates in the same way as the instrument adapter 208 of the first embodiment described above.

In the shown embodiment, the surgical instrument is a forceps, as shown in **Figs. 10-****11**.

The surgical instrument 302, here a forceps, is adapted for use in surgical, microsurgical or super-microsurgical procedures.

The surgical instrument 302 is configured for manual operation.

The surgical instrument 302 comprises a first coupling and alignment means 316.

The first coupling and alignment means 316 are formed on an external surface of the surgical instrument 302.

In the present embodiment, the first coupling and alignment means 316 are formed on the grip portion 304 of the instrument 302.

The surgical instrument adapter 308 comprises a second coupling and alignment means 318, configured for engagement with said first coupling and alignment means 316.

This allows to facilitate positioning and maintain correct placement of the surgical instrument 302 with respect to the surgical instrument adapter 308.

The second coupling and alignment means 318 is formed on an internal surface of the first movable arm 310 and/or the second movable arm 312 of the surgical instrument adapter 308.

In the present embodiment, the first coupling and alignment means 316 and the second coupling and alignment means 318 are configured for shape-coupling.

In the present embodiment, the first coupling and alignment means 316 is formed on the grip portion 304 of the surgical instrument 302, arranged at a predefined distance from the predefined workpoint W.

Similar to the first embodiment, the first coupling and alignment means 316 comprises at least one hole 320, 322, and the second coupling and alignment means 318 comprises at least one corresponding pin; 324, 326, configured for engagement with said at least one hole 320; 322.

The engagement between the first coupling and alignment means 316 and the second coupling and alignment means 318 is shown in the detail view provided in **Fig. 9**.

The first coupling and alignment means 316 comprises a first hole 320 and a second hole 322, each formed on a respective side of the surgical instrument 302, as shown in **Fig. 11**.

As mentioned, on the shown embodiment, the surgical instrument 302 is a forceps, including a first instrument arm and a second instrument arm (as best seen in **Figs. 10-11**), configured to perform an opening/closing movement with respect to each other.

Here, the first hole 320 is formed on the external surface of the grip portion part formed on the first instrument arm, while the second hole 322 is formed on the external surface of the grip portion part formed on the second instrument arm (**Fig. 11**).

The second coupling and alignment means 318 comprises a first pin 324, configured to engage with the first hole 320, and a second pin 326, configured for engagement with the second hole 322 (**Fig. 9**).

The present embodiment differs from the first embodiment above in that the first hole 320 and corresponding first pin 324 and the second hole 322 and corresponding second pin 326 have the same size.

In the present embodiment, the distance between the workpoint W of the instrument 302 and the first hole 320 and corresponding first pin 324, and the distance between the workpoint W of the instrument 302 and the second hole 322 and corresponding second pin 326 is the same.

Also, in the shown embodiment, there is no offset between the first hole 320 and the second hole 322 and, correspondingly, between the first pin 324 and the second pin 326).

The present invention further provides an end-effector system (not shown).

In particular, the end-effector system is configured for use with a surgical, microsurgical or super-microsurgical robot arm.

The end-effector system comprises an end-effector, said end-effector comprising:
an end-effector base,
a first movable finger, and
a second movable finger.

The first and second movable fingers extend distally from the end-effector base.

The system further comprises a sterile drape.

The sterile drape is configured to cover the end-effector, to maintain the sterile barrier during use.

The system further comprises the instrument-adapter assembly defined in the foregoing.

The surgical instrument adapter of the instrument-adapter assembly is arranged over the sterile drape.

Also, the surgical instrument adapter is configured to transmit a movement of a tip portion of the first and second movable fingers of the end-effector to the surgical instrument to be operated through the robot arm.

Eventually, the present invention provides a surgical, microsurgical or super-microsurgical robot arm.

In a non-limiting example, the robot arm can be used to perform anastomoses.

The robot arm includes the end-effector system defined in the foregoing.

### Reference list

- 100: Robotic system
- 102: Base station
- 104: Base column
- 106: Display module
- 108: Suspension arm
- 110: Fork-like element
- 112: (First) surgical, microsurgical or super-microsurgical robot arm
- 114: (Second) surgical, microsurgical or super-microsurgical robot arm
- 116: Surgical instrument
- 118: Surgical instrument
- 120: Wheels

- 200; 300: Instrument-adapter assembly

- 202; 302: Surgical instrument
- 204; 304: Grip portion
- 206; 306: Tip portion
- 208; 308: Surgical instrument adapter
- 210; 310: First movable arm
- 212; 312: Second movable arm
- 214: Base portion
- 216; 316: First coupling and alignment means
- 218; 318: Second coupling and alignment means
- 220, 222; 320, 322: Hole (first coupling and alignment means)
- 224, 226; 324, 326: Pin (second coupling and alignment means)

- W: Workpoint (of the surgical instrument)

- B: Operating bed
- M: Microscope
- S1: First surgeon
- S2: Second surgeon
- P: Patient
- R: Operating room

## Claims

1. An instrument-adapter assembly (200; 300) for an end-effector system, comprising:
a surgical instrument (202; 302) comprising a grip portion (204; 304), a tip portion (206; 306), distally arranged with respect to said grip portion (204; 304), and a predefined workpoint (W), and
a surgical instrument adapter (208; 308), configured to hold the surgical instrument (202; 302) and operate the surgical instrument (202; 302) by transmitting movement from an end-effector of the end-effector system to the surgical instrument (202; 302), said surgical instrument adapter (208; 308) comprising:
a first movable arm (210; 310);
a second movable arm (212; 312), and
a base portion (214),
the first movable arm (210; 310) and the second movable arm (212; 312) being hingedly connected through said base portion (214),
wherein the surgical instrument (202; 302) comprises a first coupling and alignment means (216; 316), formed on an external surface of the surgical instrument (202; 302),
wherein the surgical instrument adapter (208; 308) comprises a second coupling and alignment means (218; 318), configured for engagement with said first coupling and alignment means (216; 316), to facilitate positioning and maintain correct placement of the surgical instrument (202; 302) with respect to the surgical instrument adapter (208; 308), and
wherein the second coupling and alignment means (218; 318) is formed on an internal surface of the first movable arm (210; 310) and/or the second movable arm (212; 312) of the surgical instrument adapter (208; 308).

2. The instrument-adapter assembly (200; 300) according to claim 1, **characterized in that**
the first coupling and alignment means (216; 316) is formed on the grip portion (204; 304) of the surgical instrument (202; 302).

3. The instrument-adapter assembly (200; 300) according to claim 1 or 2, **characterized in that**
the first coupling and alignment means (216; 316) and the second coupling and alignment means (218; 318) are configured for shape-coupling.

4. The instrument-adapter assembly (200; 300) according to claim 2 or 3, **characterized in that**
the first coupling and alignment means (216; 316) is formed on the grip portion (204; 304) of the surgical instrument (202; 302), arranged at a predefined distance from the predefined workpoint (W).

5. The instrument-adapter assembly (200; 300) according to any of the preceding claims,
**characterized in that**
the first coupling and alignment means (216; 316) comprises at least one hole (220, 222; 320, 322), and
the second coupling and alignment means (218; 318) comprises at least one corresponding pin (224, 226; 324, 326), configured for engagement with said at least one hole (220, 222; 320, 322).

6. The instrument-adapter assembly (200; 300) according to claim 5, **characterized in that**
the first coupling and alignment means (216; 316) comprises a first hole (220; 320) and a second hole (222; 322), each formed on a respective side of the surgical instrument (202; 302), and
the second coupling and alignment means (218; 318) comprises a first pin (224; 324), configured for engagement with the first hole (220; 320), and a second pin (226; 326), configured for engagement with the second hole (222; 322).

7. The instrument-adapter assembly (200) according to claim 6, **characterized in that**
the first hole (220) and corresponding first pin (224) and the second hole (222) and corresponding second pin (226) have different sizes.

8. The instrument-adapter assembly (200) according to claim 7, **characterized in that**
the first hole (220) and the second hole (222) are offset with respect to each other by a predefined distance, and the first pin (224) and second pin (226) are correspondingly offset with respect to each other by the same distance.

9. The instrument-adapter assembly (300) according to claim 6, **characterized in that**
the first hole (320) and corresponding first pin (324) and the second hole (322) and corresponding second pin (326) have the same size.

10. The instrument-adapter assembly (300) according claim 9, **characterized in that**
wherein the distance between the workpoint (W) of the instrument (302) and the first hole (320) and corresponding first pin (324), and the distance between the workpoint (W) of the instrument (302) and the second hole (322) and corresponding second pin (326) is the same.

11. The instrument-adapter assembly (200; 300) according to any of the preceding claims,
**characterized in that**
the surgical instrument (202; 302) is one of a forceps-type surgical instrument or a scissors-type surgical instrument, having a first instrument arm and a second instrument arm configured to perform an opening/closing movement with respect to each other.

12. The instrument-adapter assembly (200; 300) according to claim 11, **characterized in that**
the first hole (220; 320) is formed on the external surface of the grip portion part formed on the first instrument arm, and the second hole (222; 322) is formed on the external surface of the grip portion part formed on the second instrument arm.

13. An end-effector system for a surgical, microsurgical or super-microsurgical robot arm, comprising:
an end-effector, comprising:
an end-effector base,
a first movable finger, and
a second movable finger,
said first and second movable fingers distally extending from the end-effector base;
a sterile drape, configured to cover the end-effector, and
the instrument-adapter assembly (200; 300) according to any of claims 1-10,
wherein the surgical instrument adapter (208; 308) of the instrument-adapter assembly (200; 300) is configured to transmit a movement of a tip portion of the first and second movable fingers of the end-effector to the surgical instrument (202; 302) to be operated through the robot arm.

14. A surgical, microsurgical or super-microsurgical robot arm comprising the end-effector system of claim 13.
